# EUROPEAN PATENT SPECIFICATION

(11) **EP 3 650 557 B1**
(45) Date of publication and mention of the grant of the patent: **20.10.2021**
(21) Application number: 18204853.8
(22) Date of filing: 07.11.2018
(51) Int. Cl.: C12Q 1/6823, C12N 9/22, C12Q 1/44, C12Q 1/66, C12N 15/11

(54) **METHOD FOR DETERMINATION OF RESTRICTION EFFICIENCY OF ENDONUCLEASES MEDIATING DOUBLE-STRAND BREAKS IN DNA TARGET SEQUENCES**
VERFAHREN ZUR BESTIMMUNG DER RESTRIKTIONSEFFIZIENZ VON ENDONUKLEASEN, DIE DOPPELSTRANGBRÜCHE IN DNA-ZIELSEQUENZEN VERMITTELN
PROCÉDÉ DE DÉTERMINATION DE L'EFFICACITÉ DE LA RESTRICTION D'ENDONUCLÉASES DE MÉDIATION DE CASSURES DE DOUBLES BRINS DANS DES SÉQUENCES CIBLES D'ADN

(43) Date of publication of application: 13.05.2020
(73) Proprietor: Justus-Liebig-Universität Gießen, 35390 Gießen (DE)
(72) Inventor: Stieger, Knut, 35423 Lich (DE); Lorenz, Birgit, 35321 Laubach (DE); Wimmer, Tobias, 35392 Giessen (DE)
(74) Representative: Stumpf, Peter

(56) References cited:
- WO-A2-01/68824
- US-A1- 2015 344 912
- DAVID A. HILLER ET AL: "Simultaneous DNA Binding and Bending by EcoRV Endonuclease Observed by Real-Time Fluorescence +", BIOCHEMISTRY, vol. 42, no. 49, 1 December 2003 (2003-12-01), pages 14375-14385, XP055544427, US ISSN: 0006-2960, DOI: 10.1021/bi035520w

## Description

### Background of the invention

Endonucleases, especially CRISPR (Clustered Regulatory Interspaced Short Palindromic Repeats)-Cas systems, have emerged rapidly in the past years as an effective and reliable tool for genetic engineering and genome editing. CRISPR-Cas also offers vast therapeutic potential, but an important hurdle of this technology are the off-target mutations it can induce.

Genome editing is based on the capacity of the cell to repair an induced DNA double strand break (DSB), either without the help of a template (i.e. non-homologous end-joining NHEJ) or by use of a template DNA with long (homology directed repair (HDR)) or short (microhomology mediated end-joining (MMEJ)) homologous sequences. While the NHEJ repair pathway is error-prone through the introduction of insertions and deletions (indel formation) at the DNA break site, this does not happen during HDR and MMEJ. In the absence of a template NHEJ is the predominant repair mechanism.

The DSB is induced by endonucleases, the most commonly used one being the RNA based CRISPR-Cas (clustered regularly interspaces short palindromic repeats (CRISPR) associated system) molecule. According to the state of the art, this system comprises a nuclease (e.g. the Cas9 protein) and a guide RNA (gRNA) that contains a RNA sequence complementary to the DNA target sequence which is located next to a protospacer adjacent motif (PAM) site.

By designing the guide RNA with the DNA target sequence of choice, one can very easily address almost any given DNA sequence within the genome. Other endonucleases that can be used to target DNA sequences of choice include the TALE (transcription activator like effector) and ZF (zink finger) nucleases.

An important aspect of working with the CRISPR-Cas system is careful selection of sites for the specific introduction of a DNA double-strand break. To avoid off-target effects, desired sites have to be selected under avoidance of regions of repeated sequences as well as regions with a high homology to other genome regions. Therefore, preliminary bioinformatic analyses are performed.

Different reporter systems for the determination of the induction of DSBs in given target sequences are state of the art and have been generated to validate the activity of nucleases. These reporter systems are using fluorescent or luminescent reporters for the determination of DSB events, like e.g. the Traffic Light Reporter system. The repair mechanism (HDR, MMEJ or NHEJ), which shows different activities dependent on the cell cycle phase, has to be evaluated with these reporter systems which is currently a hurdle. Another disadvantage is that the acceptance of mismatches between the target sequence and the nuclease has to be evaluated before starting genome editing. The problem with these reporter systems is that they are designed for a specific purpose and are not very sensitive. Their use is time consuming and they do not allow the use as a high-throughput assay. These qualitative, episomal assays are not able to quantify DSBs and there are no suitable controls available for the quantification of the generated DSBs.

For instance, the surveyor nuclease assay is an enzyme mismatch cleavage assay used to detect single base mismatches or small insertions or deletions (indels). Surveyor nuclease is part of a family of mismatch-specific endonucleases that were discovered in celery (CEL nucleases). The enzyme recognizes all base substitutions and insertions/deletions, and cleaves the 3' end of mismatched sites in both DNA strands with high specificity. This assay has been used to identify and analyze mutations in a variety of organisms and cell types, as well as to confirm genome modifications following genome editing using CRISPR-Cas or other endonucleases. One of the main limitations of this assay is that it relies on PCR amplification and is therefore influenced by the quality of the amplified product. PCR artifacts (e.g. primer-dimers or truncated products) can increase the background noise and obscure the signal. Primer-dimers can also inhibit the activity of surveyor nuclease, reducing the signal. As the PCR method has the potential to introduce its own mutations during the amplification, these errors can also increase background noise. Surveyor nuclease also has a 5' exonuclease activity that attacks the ends of double-stranded DNA increasing background signal during extended incubation. Because PCR and gel or capillary electrophoresis are necessary for analysis, the method is quite elaborate and expensive.

Another method is the Traffic Light Reporter system. In this system the DNA target sequence of interest is shuttle-cloned into a vector and then exposed to the CRISPR-Cas9 endonuclease. Afterwards, the resulting fluorescence signal is measured by FACS analysis and fluorescing cells are counted. Because it is unknown how many plasmids were modified by shuttle-cloning and really bear the DNA target sequence and just the fluorescence of the whole cells is generally measured, this method is also highly elaborate and not very sensitive. Furthermore, it is not suitable for high-throughput analyses.

### Technical problem

Off-target mutations are an important caveat of endonucleases used for genome editing approaches, for example the CRISPR-Cas system, that need to be addressed. Even a low frequency of unintended mutations might have deleterious effects and improving CRISPR-Cas specificity is essential for a reliable genome editing. With the aim to develop CRISPR-Cas systems comprising highly specific gRNAs that induce much less or even no off-target mutations than presently known from the state of the art, a reliable and cost-effective method for analysis of newly synthesized CRISPR-Cas systems and the DNA double-strand breaks induced by them is needed, which is furthermore suitable for high-throughput application.

### Solution of the problem

The aforementioned technical problem is solved by a method using a biosensor encoding a DNA target sequence of interest to measure the restriction efficiency of a given endonuclease system comprising a gRNA that is complementary to the DNA target sequence of the biosensor, wherein the BRET (bioluminescence resonance energy transfer) technology is involved as readout.

### Description of the invention

The invention provides a method using a biosensor for determination of restriction efficiency of endonucleases or endonuclease systems that induce DNA double-strand breaks, wherein restriction efficiency of the endonuclease at the DNA target sequence is measured by bioluminescence resonance energy transfer ratio (BRET). The scope of the invention is defined in the appended clams.

According to the present disclosure, in the first step of the method a DNA target sequence of interest is cloned into a vector encoding a BRET system for synthesis of a biosensor (Example 1).

This BRET system consists of genes encoding Luciferase 8 and GFP2 and preferably is encoded in vector pRLuc8-(Avrll/BsiWl)-GFP2. Such vectors and BRET systems are known to the skilled person.

In another preferred embodiment, for cloning of the DNA target sequence of interest into vector pRLuc8-(Avrll/BsiWl)-GFP2 the DNA target sequence is synthesized comprising restriction sites for Avrll and BsiWl at the 5'-prime and the 3'-prime end, respectively, and a PAM sequence according to standard procedures (Example 1). Protospacer adjacent motif (PAM) is a two to six base pair DNA sequence immediately following a non-self DNA sequence that is targeted by the Cas9 nuclease in the CRISPR bacterial adaptive immune system. PAM is crucial for binding of and cleavage by Cas9 and is an essential targeting component (not found in the bacterial genome) which distinguishes bacterial self DNA from non-self DNA, thereby preventing the bacterial CRISPR locus from being targeted and destroyed by the nuclease.

In a second step of the disclosure, a guide RNA (gRNA) that is complementary to the DNA target sequence of step 1 is cloned into a vector encoding an endonuclease or endonuclease system.

This endonuclease system is CRISPR-Cas9. The gRNA of interest has a complementarity to the DNA target sequence with a maximum of three mismatches and is cloned into a vector, e.g. px459, encoding CRISPR-Cas9. For cloning of the gRNA sequence of interest into vector px459 the gRNA sequence is synthesized comprising restriction site for Bbsl-HF according to standard procedures. After hybridization of the synthesized gRNA sequence, the hybridized gRNA and the vector are restricted with Bbsl-HF and then ligated via procedures that are known to a skilled person to provide a new CRISPR-Cas-gRNA plasmid (Example 2).

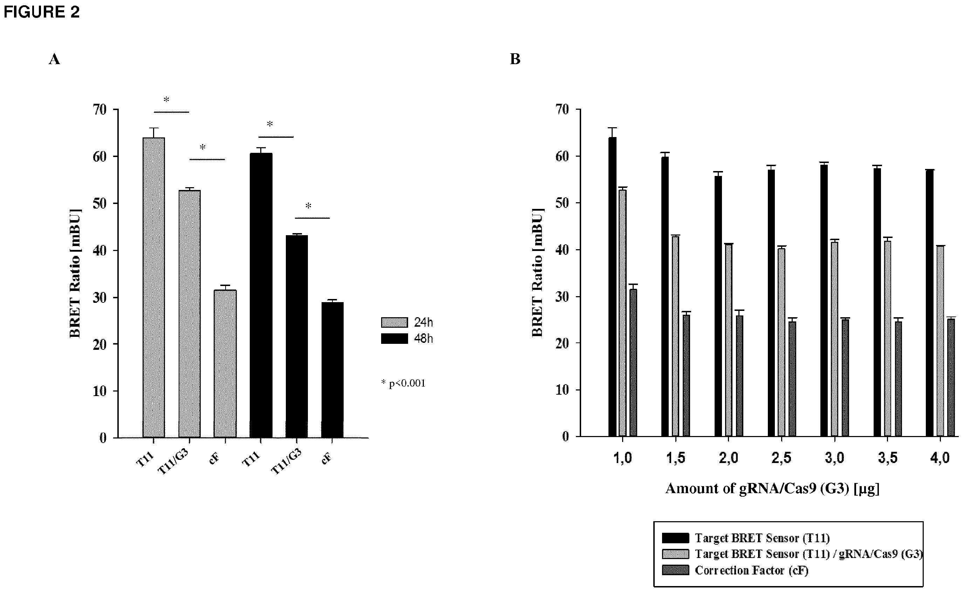

In a step of the invention, the biosensor from step 1 and the CRISPR-Cas-gRNA from step 2 are functionally analysed. Therefore, both plasmids are co-transfected according to example 3 and figure 2 into cells and the BRET ratio resulting from interaction of the CRISPR-Cas-gRNA with the DNA target sequence of the biosensor is measured after isolation of proteins from the cells following incubation for at least 24 hours according to standard procedures and addition of the substrate for the fluorescent protein RLuc8, coelenterazine 400a. The BRET ratio is the quotient of the fluorescence emission signal from the acceptor fluorophore (GFP2) and the luminescence emission signal of the donor luciferase(RLuc8) of the BRET system minus the quotient of the luminescence emission signal of the donor luciferase (RLuc8) alone as correction factor (negative control c) according to example 3).

Surprisingly it is found in the present invention that the BRET ratio corresponds to restriction efficiency of the CRISPR-Cas-gRNA that induces a DNA double-strand break (DSB) in the DNA target sequence in the biosensor of interest as well as insertions or deletions at the break site in 66% of the DSB events. Due to failure of the NHEJ repair mechanism to remove these insertions or deletions, a frameshift occurs that leads to a failure of expression of the downstream gene that encodes the acceptor fluorophore GFP2. When measuring the BRET ratio according to example 3, this can therefore directly be correlated with the restriction efficiency of the CRISPR/Cas-gRNA that has been used to induce the DSB (Figure 2). This allows for testing of every gRNA of choice in combination with every DNA target sequence of interest.

### Examples

The examples below illustrate the present invention.

### Example 1: Cloning of target sequence into BRET vector for synthesis of biosensor

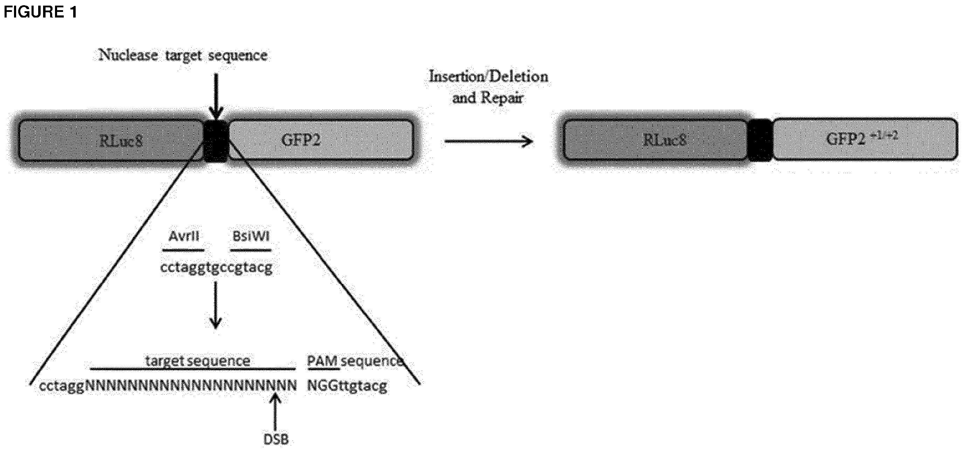

According to the present disclosure, the target sequence is cloned into vector pRLuc8-(AvrII/BsiWI)-GFP2 encoding a BRET system that is described in the state of the art. First, forward and reverse oligonucleotides encoding the target sequence of interest and Avrll or BsiWl restriction sites as well as a PAM sequence are synthesized by known methods (Figure 1, SEQ ID No. 1 and 2). For hybridization, e.g. 8 µl of the forward target sequence-oligonucleotide (100 pM) are mixed with 8 µl of the reverse target sequence-oligonucleotide (100 pM) and 4 µl of ddH₂O. The mixture is then incubated at 95°C for 10 min, followed by incubation at room temperature for 30 min. Then the hybridized target sequence-oligonucleotides and 1,000 ng of the vector pRLuc8-(Avrll/BsiWl)-GFP2 are each restricted at 37°C overnight with 1 µl of restriction enzyme Avrll and/or BsiWl under addition of 1 µl 10x CutSmart buffer and 20 µl of ddH₂O. Afterwards, the cut vector is ligated with the cut target sequence-oligonucleotide. Therefore, 100 ng of cut vector is mixed with 5 µl of the cut target sequence-oligonucleotides, 2 µl of 10x Ligase buffer, 1 µl T4-DNA Ligase (NEB, M0202S), and 20 µl of ddH₂O according to standard procedures. The ligated target sequence biosensor plasmid is then transformed into E. *coli* by heat shock or electroporation and plated on LB/Amp agar plates according to methods known from the state of the art. The new biosensor plasmid is then isolated, propagated and sequenced according to known procedures.

### Example 2: Cloning of guide RNA (gRNA) into CRISPR/Cas vector

According to the present disclosure the guide RNA (gRNA) is cloned into a vector encoding CRISPR/Cas, for example the vector px459 (pSpCas9(BB)-2A-Puro, Addgene #62988). First, forward and reverse oligonucleotides encoding the gRNA of interest and a Bbsl-HF restriction site are synthesized by known methods (SEQ ID No. 3 and 4). For hybridization, e.g. 8 µl of the forward gRNA-oligonucleotide (100 pM) are mixed with 8 µl of the reverse gRNA-oligonucleotide (100 pM) and 4 µl of ddH₂O. The mixture is then incubated at 95°C for 10 min, followed by incubation at room temperature for 30 min. Then the hybridized gRNA-oligonucleotides and 1,000 ng of the vector, e.g. px459, are each restricted at 37°C overnight with 1 µl of restriction enzyme Bbsl-HF (NEB, R3539S) under addition of 1 µl 10x NEB 2.1 buffer and 20 µl of ddH₂O. Afterwards, the cut vector is ligated with the cut gRNA-oligonucleotide. Therefore, 100 ng of cut px459 is mixed with 5 µl of the cut gRNA-oligonucleotides, 2 µl of 10x Ligase buffer, 1 µl T4-DNA Ligase (NEB, M0202S), and 20 µl of ddH₂O according to standard procedures. The ligated px459-gRNA plasmid is then transformed into E. *coli,* e.g. strain XL1-Blue, by heat shock or electroporation and plated on LB/Amp agar plates according to methods known from the state of the art. The new CRISPR/Cas-gRNA plasmid is then isolated, propagated and sequenced according to known procedures.

### Example 3: Functional analysis of biosensor and CRISPR/Cas-gRNA plasmid

According to the invention described herein, the functional analysis of the newly synthesized plasmids as described in examples 1 and 2 begins with seeding of 500,000 up to 750,000 HEK293-T cells (ATCC, CRL-3216) per well of a 6-well plate (e.g. Greiner BioOne Cellstar) in a volume of 2 ml DMEM medium (10% FBS, 4 mM L-Glutamine, 1% Penicillin/Streptomycin). The cells are incubated overnight in a humidified incubator according to standard procedures at 37°C and 5% CO₂. At the next day, the culture media are replaced by 1.5 ml fresh DMEM medium and the cells are transfected by dropwise addition of a mixture consisting of 100 µl sterile NaCl (150 mM), 200 µl PEI-mix, and a) 1,000 ng of the biosensor according to example 1 as positive control, or b) 1,000 ng of biosensor according to example 1 plus 1,000 ng of CRISPR/Cas-gRNA plasmid of example 2, or c) 1,000 ng of the vector pRLuc8-(AvrII/BsiWI)-GFP2 as negative control. After addition of the transfection mix the cells are incubated for 4 to 6 hours in a humidified incubator as described above. Then, the culture media are again replaced with 2 ml of fresh DMEM medium and incubated for another 24 to 72 hours. For protein isolation out of the cells, the culture media are discarded and the cells are washed with 1 ml 1× PBS per well. The cells are then detached from the plate by addition of Accutase or Trypsin-EDTA mix according to standard methods and entered in 100 µl 1x PBS per well, followed by two freeze/thaw cycles in liquid nitrogen. The cell lysates are afterwards cleared from cell fragments by centrifugation at 17,000 x g and 4°C for 10 min. Samples from the supernatants (20 µl each) are then pipetted into LumiPlates. 100 µl of the substrate for the luminescent protein RLuc8, coelenterazine 400a (0.01 mg; NanoLight Inc, USA), is added to each well of the LumiPlates and BRET ratios are measured according to the state of the art (Figure 2).

### Description of the drawings

**Figure 1****.** Schematic representation of the biosensor according to example 1 of the disclosure. The DNA target sequence and a PAM sequence are cloned into the plasmid using restriction enzymes Avrll and BsiWI. During a double-stand break (DSB) in the DNA target sequence, by action of CRISPR/Cas-gRNA from example 2 of the disclosure, nucleotides are inserted or deleted. In 66% of the DSB events these insertions or deletions stay when the DSB is repaired by non-homologous end-joining (NHEJ), thereby leading to a frameshift in the gene encoding GFP2 and therefore expression of GFP2 gets lost. However, the expression of the luciferase (RLuc8) is not affected.
**Figure 2****.** Analysis of activity according to example 3 of CRISPR/Cas-gRNA (G3) of example 2 in combination with the biosensor (T11) of example 1. Therefore, the target sequence is cloned according to example 1 by use of the oligonucleotides target-T11-forward (SEQ ID No. 1) and target-T11-reverse (SEQ ID No. 2), resulting in a vector named Target-BRET-Sensor (T11). The gRNA is cloned according to example 2 by use of the oligonucleotides gRNA-G3-forward (SEQ ID No. 3) and gRNA-G3-reverse (SEQ ID No. 4), resulting in a vector named gRNA/Cas9 (G3). (A) Analysis of the activity of CRISPR/Cas9-gRNA by use of the biosensor of example 1, 24 or 48 hours after transfection of T11 alone or co-transfection of both plasmids T11 and G3 of example 1 and 2 or transfection of vector pRLuc8-(Avrll/BsiWl)-GFP2 as correction factor (cF) alone into cells. (B) Analysis of CRISPR/Cas9-gRNA by use of the biosensor of example 1 according to (A) 24 hours after transfection or co-transfection, but with constant amount of the biosensor (Target-BRET-Sensor (T11); 2 µg) combined with increasing amounts of CRISPR/Cas9-gRNA (gRNA/Cas9 (G3); 1 or up to 4 µg). In (A) and (B) the BRET ratio is determined as mBU (milli BRET Units). According to Fig. 2(A), the BRET ratio is significantly reduced already 24 hours after cotransfection. According to Fig. 2(B), the restriction efficiency is dependent on the concentration of CRISPR/Cas9-gRNA, but BRET ratio does not change anymore significantly when using amounts higher than 1.5 µg.

The following sequences referred to herein are shown in the accompanying sequence listing:

### Sequence Listing

**SEQ ID No. 1:** Oligonucleotide sequence target-T11-forward 5' - ctaggCAGAGTTTCGCTGAGTTGCTTGG -3'
**SEQ ID No. 2:** Oligonucleotide sequence target-T11-reverse 5' - gtacCCAAGCAACTCAGTGAAACTCTGc-3'
**SEQ ID No. 3:** Oligonucleotide sequence gRNA-G3-forward 5' - caccCAGAGTTTCGCTGAGTTGCT -3'
**SEQ ID No. 4:** Oligonucleotide sequence gRNA-G3-reverse 5' -aaacAGCAACTCAGCGAAACTCTG-3'

## Claims

1. Method for determination of restriction efficiency of an endonuclease or endonuclease system comprising a guide-RNA that induces double-strand breaks at a DNA target sequence, wherein restriction efficiency of the endonuclease at the DNA target sequence is measured by bioluminescence resonance energy transfer ratio wherein the endonuclease or endonuclease system is CRISPR-Cas and
wherein the Cas protein is spCas9, saCas9, or Cpf1,
wherein the DNA target sequence is flanked by a sequence encoding RLuc8 and a first restriction site at its 5'prime end and a PAM sequence, a second restriction site and a sequence encoding GFP2 at its 3'prime end,
wherein the first restriction site at the 5'prime end of the DNA target sequence is Avrll and the second restriction site at the 3'prime end of the DNA target sequence is BsiWI.

2. Method according to claim 1, wherein the guide-RNA is complementary to the DNA target sequence with a maximum of three mismatches.

3. Method according to claims 1 or 2, wherein the restriction efficiency of the endonuclease or endonuclease system comprising a guide-RNA at a DNA target sequence is determined by the following steps:
- Cloning of the DNA target sequence with its flanking sequences into a plasmid.
- Cloning of a guide-RNA that is complementary to the DNA target sequence into a plasmid encoding an endonuclease or endonuclease system.
- Cotransfection of both plasmids in cell culture and coexpression for at least 24 hours.
- Isolation of proteins from cell culture and stimulation of bioluminescence resonance energy transfer by addition of the luciferase substrate coelenterazine.
- Determination of bioluminescence resonance energy transfer ratio by measurement of the quotient of the fluorescence emission signal from the acceptor fluorophore GFP2 and the luminescence emission signal of the donor luciferase RLuc8 minus the quotient of the luminescence emission signal of the donor luciferase RLuc8 alone as correction factor.

## Patentansprüche

1. Verfahren zur Bestimmung der Restriktionseffizienz einer Endonuklease oder eines Endonuklease-Systems, umfassend eine guide-RNA, die Doppelstrangbrüche an einer DNA-Zielsequenz induziert, wobei die Restriktionseffizienz der Endonuklease an der DNA-Zielsequenz durch das Biolumineszenz-Resonanz-Energie-Transfer-Verhältnis gemessen wird, wobei die Endonuklease oder das Endonuklease-System CRISPR-Cas ist und wobei das Cas-Protein spCas9, saCas9 oder Cpf1 ist, wobei die DNA-Zielsequenz von einer Sequenz, die RLuc8 kodiert, und einer ersten Restriktionsstelle an ihrem 5'Prime-Ende und einer PAM-Sequenz, einer zweiten Restriktionsstelle und einer Sequenz, die GFP2 kodiert, an ihrem 3'Prime-Ende flankiert ist, wobei die erste Restriktionsstelle am 5'Prime-Ende der DNA-Zielsequenz Avrll ist und die zweite Restriktionsstelle am 3'Prime-Ende der DNA-Zielsequenz BsiWI ist.

2. Verfahren nach Anspruch 1, wobei die guide-RNA komplementär zur DNA-Zielsequenz mit maximal drei Fehlpaarungen ist.

3. Verfahren nach Anspruch 1 oder 2, wobei die Restriktionseffizienz der Endonuklease oder des Endonuklease-Systems, das eine guide-RNA enthält, an einer DNA-Zielsequenz durch die folgenden Schritte bestimmt wird:
- Klonierung der DNA-Zielsequenz mit ihren flankierenden Sequenzen in ein Plasmid.
- Klonierung einer zur DNA-Zielsequenz komplementären guide-RNA in ein Plasmid, das für eine Endonuklease oder ein Endonuklease-System kodiert.
- Cotransfektion beider Plasmide in Zellkultur und Koexpression für mindestens 24 Stunden.
- Isolierung der Proteine aus der Zellkultur und Stimulierung des Biolumineszenz-Resonanz-Energie-Transfers durch Zugabe des Luciferase-Substrats Coelenterazin.
- Bestimmung des Biolumineszenz-Resonanz-Energie-Transfer-Verhältnisses durch Messung des Quotienten aus dem Fluoreszenz-Emissionssignal des Akzeptor-Fluorophors GFP2 und dem Lumineszenz-Emissionssignal der Donor-Luciferase RLuc8 abzüglich des Quotienten aus dem Lumineszenz-Emissionssignal der Donor-Luciferase RLuc8 allein als Korrekturfaktor.

## Revendications

1. Procédé de détermination de l'efficacité de restriction d'une endonucléase ou d'un système d'endonucléase comprenant un guide-ARN qui induit des cassures du double-brin au niveau d'une séquence d'ADN cible, dans lequel l'efficacité de restriction de l'endonucléase au niveau de la séquence cible d'ADN est mesurée par un rapport de transfert d'énergie de résonance de bioluminescence, dans lequel l'endonucléase ou le système d'endonucléase est CRISPR-Cas et dans lequel la protéine Cas est spCas9, saCas9, ou Cpf1, dans laquelle la séquence d'ADN cible est flanquée d'une séquence codant pour RLuc8 et d'un premier site de restriction à son extrémité 5-prime et d'une séquence PAM, d'un second site de restriction et d'une séquence codant pour GFP2 à son extrémité 3-prime,
où le premier site de restriction à l'extrémité 5-prime de la séquence cible d'DNA est AvrII et le second site de restriction à l'extrémité 3-prime de la séquence d'ADN cible est BsiWI.

2. Procédé selon la revendication 1, dans lequel l'ARN-guide est complémentaire de la séquence d'ADN cible avec un maximum de trois désappariements.

3. Procédé selon les revendications 1 ou 2, dans lequel l'efficacité de restriction de l'endonucléase ou du système d'endonucléase comprenant un guide-ARN au niveau d'une séquence d'ADN cible est déterminée par les étapes suivantes:
- Clonage de la séquence d'ADN cible avec ses séquences flanquantes dans un plasmide.
- Clonage d'un ARN-guide complémentaire de la séquence d'ADN cible dans un plasmide codant pour une endonucléase ou un système d'endonucléase.
- Cotransfection des deux plasmides dans une culture cellulaire et coexpression pendant au moins 24 heures.
- Isolement des protéines de la culture cellulaire et stimulation du transfert d'énergie par résonance de bioluminescence par l'ajout de la coelentérazine, substrat de la luciférase.
- Détermination du rapport de transfert d'énergie par résonance de bioluminescence par la mesure du quotient du signal d'émission de fluorescence du fluorophore accepteur GFP2 et du signal d'émission de luminescence de la luciférase donneuse RLuc8 moins le quotient du signal d'émission de luminescence de la luciférase donneuse RLuc8 seule comme facteur de correction.


### SEQUENCE LISTING

<110> Justus-Liebig-Universität
<120> Method for determination of restriction efficiency of endonucleases
   mediating double-strand breaks in DNA target sequences
<130> EP
<160> 4
<170> BiSSAP 1.3.5
<210> 1
   <211> 28
   <212> DNA
   <213> Artificial Sequence
<223> "oligonucleotide sequence "
<220>
   <223> oligonucleotide sequence
<400> 1
   ctaggcagag tttcgctgag ttgcttgg 28
<210> 2
   <211> 28
   <212> DNA
   <213> Artificial Sequence
<223> "oligonucleotide sequence"
<220>
   <223> oligonucleotide sequence
<400> 2
   gtacccaagc aactcagtga aactctgc 28
<210> 3
   <211> 24
   <212> DNA
   <213> Artificial Sequence
<223> "oligonucleotide sequence"
<220>
   <223> oligonucleotide sequence
<400> 3
   cacccagagt ttcgctgagt tgct 24
<210> 4
   <211> 24
   <212> DNA
   <213> Artificial Sequence
<223> "oligonucleotide sequence"
<220>
<223> oligonucleotide sequence
<400> 4
   aaacagcaac tcagcgaaac tctg 24